# EUROPÄISCHE PATENTSCHRIFT

(11) **EP 1 904 023 B1**
(45) Veröffentlichungstag und Bekanntmachung des Hinweises auf die Patenterteilung: **31.12.2014**
(21) Anmeldenummer: 06764103.5
(22) Anmeldetag: 07.07.2006
(51) Int. Cl.: A61K 8/06, A61K 8/81, A61K 8/73, A61Q 19/06, A61K 8/44

(54) **EMULGATORKOMBINATION FÜR KOSMETIKA**
EMULSIFIER COMBINATION FOR COSMETICS
ASSOCIATION D'EMULSIFIANTS POUR PRODUITS COSMETIQUES

(30) Priorität: 07.07.2005 DE 102005032237
(43) Veröffentlichungstag der Anmeldung: 02.04.2008
(73) Patentinhaber: Beiersdorf AG, 20253 Hamburg (DE)
(72) Erfinder: SCHULZ, Jens, 22869 Schenefeld (DE); NIELSEN, Jens, 25448 Henstedt-Ulzburg (DE); KRÖPKE, Rainer, 22869 Schenefeld (DE); HAMER, Gunhild, 22455 Hamburg (DE); HEPTNER, Astrid, 20257 Hamburg (DE); BEHRENS, Svea, 21109 Hamburg (DE)
(86) Internationale Anmeldenummer: PCT/EP2006/064003
(87) Internationale Veröffentlichungsnummer: WO 2007/006745

(56) Entgegenhaltungen:
- DE-A- 10 143 964
- DE-A1- 4 401 308
- FR-A- 2 758 724
- "International Cosmetic Ingredient Dictionary and Handbook" 2004, THE COSMETIC, TOILETRY, AND FRAGRANCE ASSOCIATION , XP002400789 Seite 32 Seite 287 - Seite 288 Seite 359 Seite 1761 Seite 1982

## Beschreibung

Die vorliegende Erfindung betrifft eine kosmetische Zubereitung enthaltend eine Emulgatorkombination aus Polyacrylsäuresalzen, Acrylat/C10-30 Alkylacrylat Crosspolymer und Carrageenan und die Verwendung der kosmetischen Zubereitung.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Die Haut benötigt fetthaltige und wasserlösliche Pflegestoffe. Daher enthalten Hautpflegemittel heutzutage in der Regel einen Öl/Fettkörper und eine Wasserphase.

Fette und Öle sind von Natur aus mit Wasser und wasserlöslichen Stoffen (z.B. Salze) nicht mischbar. Um aus Wasser und Ölphase eine optisch homogene, stabile Zubereitung zu erhalten, müssen Ihnen grenzflächenaktive Zusatzstoffe (Emulgatoren) zugesetzt werden, die in der Lage sind kleinste Öl- und Feststoffkügelchen in Wasser (Öl-in-Wasser-Emulsion, ONV-Emulsion) oder Wassertröpfchen in Ölen (Wasser-in-Öl-Emulsion, W/O-Emulsion) zu verteilen. Die entstandenen Emulsionen erlauben eine "2 in 1"-Pflege der Haut mit wasser- und öllöslichen Stoffen. Je nach Konsistenz werden sie als Salben oder Cremes (zähflüssig bis schnittfest) oder Lotionen (dünnflüssig) bezeichnet.

Emulsionen haben hervorragende kosmetische Eigenschaften: Die lipophilen Bestandteile ziehen relativ schnell in die Haut ein, die wässrigen Bestandteile erhöhen nachhaltig die Hautfeuchte.

Die Eigenschaften einer kosmetischen Emulsion sind ganz wesentlich von den eingesetzten Emulgatoren abhängig. Zu den ältesten bekannten Emulgatoren gehören die Seifen wie beispielsweise Natriumstearat.

Emulgatoren und Emulgatorsysteme des Standes der Technik weisen eine Reihe von Nachteilen auf:
- Die Emulgatoren müssen Zubereitungsbestandteile unterschiedlichster Zusammensetzung und mit unterschiedlichen Eigenschaften wie Polarität (bei z.B. Ölen) oder lonenstärke (bei salzartigen Bestandteile der wässrigen Phase) schnell und über einen längeren Zeitraum stabil zu einer optisch homogenen Emulsion emulgieren. Um diese Anforderungen zu erfüllen, muss das Emulgatorsystem häufig auf die Zusammensetzung der Emulsion individuell abgestimmt werden.
- Die Emulgatoren sollen eine gute Hautverträglichkeit aufweisen und die Haut nicht reizen.
- Die Emulgatoren sollen nicht in die Haut penetrieren.

Es war daher die Aufgabe der vorliegenden Erfindung, die Mängel des Standes der Technik zu beseitigen und ein verbessertes Emulgatorsystem zu entwickeln, welches diese Anforderungen erfüllt.

Herkömmliche Emulsionen bestehen aus einer Wasser- sowie einer Lipidphase. Es gibt einen großen Bedarf an Zubereitungen, die besonders "leicht" und fettarm sind und allenfalls geringe Mengen an Lipiden enthalten. In derartige Zubereitungen können nach dem Stand der Technik jedoch keine (oder allenfalls unzureichende Mengen) an lipophilen Wirkstoffen eingearbeitet werden.

Es war daher die Aufgabe der vorliegenden Erfindung, eine "fettarme" (d.h. weitgehend frei von Lipiden aller Art) kosmetische Zubereitung zu entwickeln, in welche sich größere Mengen an lipophilen Wirkstoffen einarbeiten lassen.

Ferner besteht der Wunsch, durch die Applikation von Kosmetika den natürlichen Fetthaushalt der Haut nicht aus dem Gleichgewicht zu bringen.

Es war daher die Aufgabe der vorliegenden Erfindung, eine kosmetische Zubereitung zu entwickeln, in die größere Mengen an lipophilen Wirkstoffen eingearbeitet sind, welche aber den natürlichen Fetthaushalt der Haut nicht aus dem Gleichgewicht bringt.

Überraschend gelöst werden die Aufgaben durch eine kosmetische Zubereitung enthaltend
a) 0,01 bis 1,5 Gewichts-% Polyacrylsäuresalzen,
b) 0,01 bis 1,5 Gewichts-% quervernetzten Polyacrylsäuresalzen,
c) 0,01 bis 1,5 Gewichts-% Acrylat/C10-30 Alkylacrylat Crosspolymer,
d) 0,01 bis 2,5 Gewichts-% Carrageenan,
wobei sich die Gewichtsangaben auf das Gesamtgewicht der kosmetischen Zubereitung beziehen.

Zwar kennt der Fachmann die DE 10143964, FR 2758724 und "International Cosmetic Ingredient Dictionary and Handbook" 2004, The Cosmetic, Toiletry, and Fragrance Association, XP002400789, doch konnten diese Schriften nicht den Weg zur vorliegenden Erfindung weisen.

Das erfindungsgemäß bevorzugte Polyacrylsäuresalz ist dabei das Natriumpolyacrylat (CAS: 9003-04-7) welches beispielsweise von der Firma Cognis unter dem Handelsnamen COSMEDIA SP angeboten wird. Diese Polyacrylsäuresalze sind nicht vernetzt.

Es ist erfindungsgemäß vorteilhaft, als quervernetzte Polyacrylsäuresalze Carbomere einzusetzen. Das erfindungsgemäß bevorzugte quervernetzte Polyacrylsäuresalz ist dabei das Natriumcarbomer (z.B: CAS: 9007-16-3, CAS:9007-17-4, CAS:9062-04-8, CAS:76050-42-5) welches beispielsweise von der Firma Noveon unter dem Handelsnamen Carbopol 980 angeboten wird.

Ein erfindungsgemäß bevorzugtes Acrylat/C10-30 Alkylacrylat Crosspolymer (INCI: Acrylates/C10-30 Alkylacrylates Crosspolymer) wird beispielsweise von der Firma Noveon unter dem Handelsnamen Pemulen TR-1 angeboten.

Das erfindungsgemäß bevorzugte Carrageenan ist unter der CAS-Nr.: 9000-07-1 (EINECS-Nr.: 232-524-2) abgelegt und kann beispielsweise unter dem Handelsnamen Gelcarin GP-379NF bei der Firma Interorgana erworben werden.

Erfindungsgemäß vorteilhafte Ausführungsformen der erfindungsgemäßen kosmetischen Zubereitung sind dadurch gekennzeichnet, dass sie als weitere Komponente 0,01 bis 2,5

Gewichts-% Vinylpyrrolidon/Vinylacetat-Copolymer (VPNA-Copolymer), bezogen auf das Gesamtgewicht der kosmetischen Zubereitung enthält.

Das erfindungsgemäß bevorzugte Vinylpyrrolidon/Vinylacetat-Copolymer (VPNA-Copolymer) ist ein Polymer, welches zu 60% aus Vinylpyrrolidon und zu 40% aus Vinylacetat gebildet wird und beispielsweise unter dem Handelsnamen Luviskol VA64 W von der Firma BASF angeboten wird.

Die erfindungsgemäße Zubereitung ist erfindungsgemäß vorteilhaft dadurch gekennzeichnet, dass das Gewichtsverhältnis von Polyacrylsäuresalzen zu Acrylates/C10-30 Alkylacrylates Crosspolymer von 1 :10 bis 10 : 1 und bevorzugt von 1 :1 bis 2 :1 beträgt.

Die erfindungsgemäße Zubereitung ist erfindungsgemäß vorteilhaft dadurch gekennzeichnet, dass das Gewichtsverhältnis von Polyacrylsäuresalzen zu Carrageenan von 1 :10 bis 10 :1 beträgt.

Die erfindungsgemäße Zubereitung ist erfindungsgemäß vorteilhaft dadurch gekennzeichnet, dass das Gewichtsverhältnis von Polyacrylsäuresalzen zu VinylpyrrolidonA/inylacetat-Copolymeren von 1:10 bis 10 :1 und bevorzugt von 1:1 bis 2 :1 beträgt.

Erfindungsgemäß ist auch eine kosmetische Zubereitung, welche die erfindungsgemäße Emulgatorkombination aus Polyacrylsäuresalzen, quervemetzten Polyacrylsäuresalzen, AcrylatlC10-30 Alkylacrylat Crosspolymer und Carageenan in einer Gesamtkonzentration von 0,5 bis 1,8 Gewichts-%, bezogen auf das Gesamtgewicht der kosmetischen Zubereitung, enthält.

Dabei handelt es sich erfindungsgemäß bevorzugt bei der kosmetischen Zubereitung um ein wässriges oder wässrig-alkoholisches Gel oder eine Gelcreme.

Gelcremes sind besonders leichte Produkte mit einem niedrigen Emulgator- und Lipidgehalt. Sie zeichnen sich dadurch aus, daß sie sich leicht auf der Haut verteilen lassen und ein Frischegefühl vermitteln. Nach dem Produktauftrag soll auf der Haut kein oder nur wenig Rückstand verbleiben. Gelcremes enthalten in der Regel einen relativ hohen Anteil an hydrophilen Verdickungsmitteln. Da sich der Verdicker oder das Verdickersystem in der äußeren Phase befindet, hat es einen signifikanten Einfluß auf die sensorischen Eigenschaften des Produktes. Gängige Verdickersysteme lassen sich entweder nicht leicht verteilen, ergeben kein Frischegefühl oder hinterlassen einen zu klebrigen Rückstand auf der Haut.

Die erfindungsgemäße kosmetische Zubereitung ist erfindungsgemäß vorteilhaft dadurch gekennzeichnet, dass die Zubereitung einen oder mehrere der Wirkstoffe gewählt aus der Gruppe der Verbindungen hydrophilen Wirkstoffe enthält, wobei ein oder mehrere Wirkstoffe aus der Gruppe der Verbindungen Camithin, Ubichinon Q10, alpha-Glucosylrutin, Kreatin, Kreatinin, Koffein, Flavonoide, Folsäure und 8-Hexadecen-1,16-dicarbonsäure (Dioic acid, CAS-Nummer 20701-68-2; vorläufige INCI-Bezeichnung Octadecendioic acid) erfindungsgemäß bevorzugt eingesetzt werden.

Derartige erfindungsgemäße wirkstoffhaltige kosmetische Zubereitungen enthalten die Wirkstoffe erfindungsgemäß vorteilhaft in einer Gesamtkonzentration von 0,0001 bis 12,5 Gewichts-% und bevorzugt in einer Gesamtkonzentration von 0,01 bis 0,8 Gewichts-%, jeweils bezogen auf das Gesamtgewicht der Zubereitung.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte und/oder Wirkstoffe wie Selbstbräuner, Repellentien, Vitamine, Pflanzenextrakte, Niacinamid, Panthenol.

Erfindungsgemäß bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Erfindungsgemäß vorteilhaft kann die erfindungsgemäße Zubereitung weitere kosmetische Zusatzstoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Die erfindungsgemäße kosmetischen Zubereitung enthält erfindungsgemäß vorteilhaft mindestens einen wasserlöslichen Farbstoff.

Die erfindungsgemäße kosmetischen Zubereitung weist erfindungsgemäß vorteilhaft ein transparentes oder transluzentes Aussehen auf. Sie kann erfindungsgemäß vorteilhaft in einer transparenten Verpackung aufbewahrt werden.

Es ist vorteilhaft im Sinne der vorliegenden Erfindung, wenn die erfindungsgemäße kosmetische Zubereitung frei ist von Tensiden mit einem HLB-Wert von größer 25. Es ist erfindungsgemäß bevorzugt, wenn die erfindungsgemäße kosmetische Zubereitung frei ist von Tensiden mit einem HLB-Wert von größer 20. Die HLB-Werte von Tensiden können den üblichen Tabellenwerken (z.B. Fiedler, Lexikon der Hilfsstoffe für Pharmazie, Kosmetik und angrenzende Gebiete, Editio Cantor Verlag, Aulendorf) entnommen werden.

Erfindungsgemäß vorteilhaft ist auch eine Emulsion aus einer kosmetischen Zubereitung (bevorzugt aus einem wässrigen oder wässrig-alkoholischem Gel) und hauteigenen Lipiden. Eine derartige Emulsion wird dabei dadurch hergestellt, dass die kosmetische Zubereitung, (bevorzugt aus einem wässrigen und wässrig-alkoholischem Gel oder Gelcreme) auf die Haut aufgetragen und anschließend verrieben wird.

Das Verfahren zur Herstellung einer derartigen Emulsion, welches dadurch gekennzeichnet ist, dass eine erfindungsgemäße kosmetische Zubereitung (bevorzugt aus einem wässrigen oder wässrig-alkoholischem Gel oder Gelcreme) auf die Haut aufgetragen und anschließend verrieben wird, ist erfindungsgemäß vorteilhaft. Dabei wird das Verreiben der kosmetischen Zubereitung auf der Haut bevorzugt mit den Händen oder Fingern durchgeführt, kann jedoch auch mit Hilfe eines Applikators (beispielsweise eines Schwämmchens oder eines Massagekopfes) erfindungsgemäß erfolgen.

Die Zubereitung kann erfindungsgemäß vorteilhaft in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Erfindungsgemäß vorteilhaft ist die Verwendung einer erfindungsgemäßen kosmetischen Zubereitung oder einer aus ihr hergestellten Emulsion zur kosmetischen Prophylaxe oder kosmetischen Behandlung von Hautunebenheiten, und/oder Cellulite.

Erfindungsgemäß vorteilhaft ist die Verwendung einer erfindungsgemäßen kosmetischen Zubereitung zur Herstellung einer Emulsion mit hauteigenen Lipiden zur kosmetischen Prophylaxe oder kosmetischen Behandlung von Hautunebenheiten und/oder Cellulite.

Unter Prophylaxe und Behandlung wird im Rahmen dieser Offenbarung ausschließlich die kosmetische Prophylaxe und Behandlung verstanden und keinesfalls eine therapeutische Prophylaxe und Behandlung im Sinne des Patentrechts.

Die erfindungsgemäße kosmetische Prophylaxe oder kosmetische Behandlung von Cellulite erfolgt erfindungsgemäß vorteilhaft in Kombination mit Massagegeräten, Ultraschall, Infrarotlicht ,Textilien, Bandagen und/oder okklusiven Folien.

Erfindungsgemäß ist nicht zuletzt die Verwendung der erfindungsgemäßen kosmetischen Zubereitung als Saunaprodukt.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

### Gele

| | **1** | **2** | **3** | **4** | **5** |
|---|---|---|---|---|---|
| Polyacrylsäure, Na-Salz (Carbomer) | 1,0 | 0,2 | 0,5 | 0,1 | 1,5 |
| PEG-40-Stearat | 0,5 | --- | 0,75 | --- | -- |
| Carrageenan | 1,25 | 0,2 | 0,2 | 0,1 | 2,5 |
| Natrium polyacrylat | 0,2 | 0,2 | 0,3 | 0,5 | 1,5 |
| Vinylpynrolidon/Vinylacetat-Copolymeren | 1,0 | --- | 0,2 | --- | --- |
| Acrylates/C10-C30 Alkyl Acrylate Crosspolymer | 1,5 | 0,2 | 0,15 | 1,5 | 0,75 |
| wasserlösliche Farbe | 0,01 | 0,4 | --- | --- | --- |
| Cyclomethicon | 1 | 8,5 | 5 | 0,5 | 3 |
| Dimethiconol | 0,5 | 0,5 | 0,75 | 1,25 | 1,0 |
| Cetylstearylalkohol | --- | --- | 1 | 1 | --- |
| hydriertes Polyisobuten | 0,5 | --- | 1,0 | --- | 0,25 |
| Camitin | 0,5 | 0,1 | 0,75 | 3,0 | 0,25 |
| Panthenol | 0,5 | --- | 0,75 | 0,25 | 0,1 |
| Glycerin | 3,0 | 8,6 | 12,5 | 17,2 | 5,0 |
| Parfum | q,s, | q,s, | q,s, | q,s, | q,s, |
| Methylpropandiol | 4,5 | 2,0 | 0,5 | 1,5 | --- |
| Propylparaben | 0,3 | --- | 0,25 | 0,15 | --- |
| Ethanol | --- | 10,0 | 5,0 | --- | 3,5 |
| Wasser | ad 100 | ad 100 | ad 100 | ad 100 | ad 100 |

## Patentansprüche

1. Kosmetische Zubereitung enthaltend
a) 0,01 bis 1,5 Gewichts-% Polyacrylsäuresalze,
b) 0,01 bis 1,5 Gewichts-% quervernetzten Polyacrylsäuresalze,
c) 0,01 bis 1,5 Gewichts-% Acrylat/C10-30 Alkylacrylat Crosspolymer, und
d) 0,01 bis 2,5 Gewichts-% Carrageenan,
wobei sich die Gewichtsangaben auf das Gesamtgewicht der kosmetischen Zubereitung beziehen.

2. Kosmetische Zubereitung nach Anspruch 1, **dadurch gekennzeichnet, dass** sie als weitere Komponente 0,01 bis 2,5 Gewichts-% Vinylpyrrolidon/ Vinylacetat-Copolymer (VPNA-Copolymer), bezogen auf das Gesamtgewicht der kosmetischen Zubereitung enthält.

3. Kosmetische Zubereitung nach Anspruch 1 oder 2, **dadurch gekennzeichnet, dass** die Emulgatorkombination aus Polyacrylsäuresalzen, quervernetzten Polyacrylsäuresalzen, Acrylat/C10-30 Alkylacrylat Crosspolymer und Carageenan in einer Gesamtkonzentration von 0,5 bis 1,8 Gewichts-%, bezogen auf das Gesamtgewicht der kosmetischen Zubereitung, enthalten ist.

4. Kosmetische Zubereitung nach einem der vorhergehenden Ansprüche,
**dadurch gekennzeichnet, dass** es sich bei der Zubereitung um ein wässriges oder wässrigalkoholisches Gel oder eine Gelcreme handelt.

5. Kosmetische Zubereitung nach einem der vorhergehenden Ansprüche,
**dadurch gekennzeichnet, dass** die Zubereitung einen oder mehrere Wirkstoffe gewählt aus der Gruppe Carnithin, Ubichinon Q10, alpha-Glucosylrutin, Kreatin, Kreatinin, Koffein, Flavonoide, Folsäure und 8-Hexadecen-1,16-dicarbonsäure in Gesamtkonzentration von 0,0001 bis 12,5 Gewichts-% , auf das Gesamtgewicht der Zubereitung enthält.

6. Kosmetischen Zubereitung nach einem der vorhergehenden Ansprüche,
**dadurch gekennzeichnet, dass** sie ein transparentes oder transluzentes Aussehen aufweist.

7. Kosmetischen Zubereitung nach einem der vorhergehenden Ansprüche,
**dadurch gekennzeichnet, dass** sie mindestens einen wasserlöslichen Farbstoff enthält.

8. Kosmetische Zubereitung nach einem der vorhergehenden Ansprüche,
**dadurch gekennzeichnet, dass** die Zubereitung frei ist von Tensiden mit einem HLB-Wert von größer 25.

## Claims

1. Cosmetic preparation comprising
a) 0.01 to 1.5% by weight of polyacrylic acid salts,
b) 0.01 to 1.5% by weight of crosslinked polyacrylic acid salts,
c) 0.01 to 1.5% by weight of acrylate/C10-30 alkyl acrylate crosspolymer, and
d) 0.01 to 2.5% by weight of carrageenan,
where the weight data refer to the total weight of the cosmetic preparation,

2. Cosmetic preparation according to Claim 1, **characterized in that** it comprises, as further component, 0.01 to 2.5% by weight of vinylpyrrolidone/vinyl acetate copolymer (VPNA copolymer), based on the total weight of the cosmetic preparation.

3. Cosmetic preparation according to Claim 1 or 2, **characterized in that** the emulsifier combination of polyacrylic acid salts, crosslinked polyacrylic acid salts, acrylate/C10-30 alkyl acrylate crosspolymer and carrageenan is present in a total concentration from 0.5 to 1.8% by weight, based on the total weight of the cosmetic preparation.

4. Cosmetic preparation according to one of the preceding claims, **characterized in that** the preparation is an aqueous or an aqueous-alcohafic gel or a gel cream.

5. Cosmetic preparation according to one of the preceding claims, **characterized in that** the preparation comprises one or more active ingredients selected from the group carnitine, ubiquinone Q10, alpha-glucosylrutin, creatine, creatinine, caffeine, flavonoids, folio acid and 8-hexadecene-1,16-dicarboxylic acid in total concentration of 0.0001 to 12.5% by weight, based on the total weight of the preparation.

6. Cosmetic preparation according to one of the preceding claims, **characterized in that** it has a transparent or translucent appearance.

7. Cosmetic preparation according to one of the preceding claims, **characterized in that** it comprises at least one water-soluble dye.

8. Cosmetic preparation according to one of the preceding claims, **characterized in that** the preparation is free from surfactants with an HLB value of greater than 25.

## Revendications

1. Composition cosmétique contenant
a) 0,01 à 1,5% en poids de sels de poly(acide acrylique),
b) 0,01 à 1,5% en poids de sels de poly(acide acrylique) réticulés,
c) 0.01 à 1,5% en poids de polymère réticulé d'acrylate/acrylate de C₁₀₋₃₀-alkyle, et
d) 0,01 à 2,5% en poids de carraghénane,
où les indications en poids se rapportent au poids total de la composition cosmétique.

2. Composition cosmétique selon la revendication 1, **caractérisée en ce qu'**elle contient, comme autre composant, 0,01 a 2,5% en poids de copolymère de vinylpyrrolidonelacétate de vinyle (copolymère VPNA), par rapport au poids total de la composition cosmétique.

3. Composition cosmétique selon la revendication 1 ou 2, **caractérisée en ce qu'**elle contient la combinaison d'émulsifiants constitués par des sels de poly(ar-ide acrylique), des sels de poly(acide acrylique) réticulés, de copolymère réticulé d'acrylate/acrylate de C₁₀-₃₀-alkyle et de carraghénane en une concentration totale de 0,5 à 1,8% en poids, par rapport au poids total de la composition cosmétique.

4. Composition cosmétique selon l'une quelconque des revendications précédentes, **caractérisée en ce qu'**il s'agit, pour la composition, d'un gel aqueux ou aqueux-alcoolique ou d'un gel-créme.

5. Composition cosmétique selon l'une quelconque des revendications précédentes, **caractérisée en ce que** la composition contient une ou plusieurs substances actives choisies dans le groupe formé par la carnitine, l'ubiquinone Q10, l'alpha-gluaosylrutine, la créatine, la créatinine, la caféine, les flavonoïdes, l'acide folique et l'acide 8-hexadécène-1,16-dicarboxylique en une concentration totale de 0,0001 à 12,5% en poids par rapport au poids total de la composition.

6. Composition cosmétique selon l'une quelconque des revendications précédentes, **caractérisée en ce qu'**elle présente un aspect transparent ou translucide.

7. Composition cosmétique selon l'une quelconque des revendications précédentes, **caractérisée en ce qu'**elle contient au moins un colorant soluble dans l'eau,

8. Composition cosmétique selon l'une quelconque des revendications précédentes, **caractérisée en ce que** la composition est exempte d'agents tensioactifs présentant une valeur BHL supérieure à 25.
